# EUROPÄISCHE PATENTANMELDUNG

(11) **EP 4 603 830 A1**
(43) Veröffentlichungstag der Anmeldung: **20.08.2025**
(21) Anmeldenummer: 24157948.1
(22) Anmeldetag: 15.02.2024
(51) Int. Cl.: G01N 27/26, G01N 33/00, G01D 11/24

(54) **GASDETEKTOR**

(71) Anmelder: Minimax Viking Patent Management GmbH, 23840 Bad Oldesloe (DE)
(72) Erfinder: Dittmer, Hauke, 23840 Bad Oldesloe (DE); Mitzlaff, Thorsten, 23840 Bad Oldesloe (DE); Kuhn, Arthur, 23840 Bad Oldesloe (DE)
(74) Vertreter: Eisenführ Speiser

(57) **Zusammenfassung**

Die vorliegende Erfindung betrifft einen Gasdetektor (100), der einen elektrochemischen Sensor (101), EC-Sensor, der zur Detektion einer Konzentration wenigstens eines Gases oder Gasgemischs ausgebildet ist, wobei der EC-Sensor (101) mehrere Elektroden (308, 310, 312), die auf einer Seite (305) des EC-Sensors (201, 301) ausgebildet sind, und einen Gaseingang aufweist, eine Sensorplatine (140), die in Verbindung mit den mehreren Elektroden des EC-Sensors (101) steht, und ein Detektorgehäuse (110), das den EC-Sensor (101) und die Sensorplatine (140) umschließt, aufweist, wobei das Detektorgehäuse (110) einen Gaseinlass (120) mit einer Einlassöffnung (121) aufweist, wobei das Detektorgehäuse (110) ein Gasausgleichsvolumen (V) im Inneren zwischen Detektorgehäuse (110) und EC-Sensor (101) definiert, das eine Gasströmung zu dem Gaseingang des EC-Sensors (101) ermöglicht.

## Beschreibung

Die Erfindung betrifft einen Gasdetektor zur Detektion einer Konzentration wenigstens eines Gases oder Gasgemischs.

Im Bereich der aktiven Brandvermeidung werden Sauerstoffreduzierungssysteme eingesetzt. Üblicherweise wird durch Zufuhr eines Inertgases, meistens Stickstoff, der Sauerstoffanteil in der Luft des vom Sauerstoffreduzierungssystem überwachten Raumes von fast 21 Vol.-% (natürliche Umgebungsluft) auf den eine Verbrennungsreaktion unmöglich machenden Anteil gezielt gesenkt. Wie stark der Gehalt an Sauerstoff gesenkt werden muss, hängt von den vorhandenen Materialien ab. Dieser Sauerstoffgehalt, Entzündungsgrenze genannt, wird für die jeweiligen Materialien unter festgelegten Prüfbedingungen ermittelt. Durch die permanente kontrollierte Zuführung von Stickstoff hält das aktive Brandvermeidungssystem den Sauerstoffgehalt der Luft im Schutzbereich dauerhaft auf gesenktem Niveau. So entsteht eine Atmosphäre in der sich Brände nicht entwickeln können.

Für solche Sauerstoffreduzierungssysteme werden zuverlässige und zügig reagierende Sauerstoffdetektoren benötigt. Der Sauerstoffgehalt, O2-Gehalt, in den überwachten Räumen (bspw. Rechenzentren) wird ständig gemessen, um sicherzustellen, dass er sich ständig innerhalb vorgegebener Konzentrationsgrenzen bewegt. Durch grundsätzliche Undichtigkeit der Räume gibt es einen stetigen Eintrag von Außenluft, so dass immer wieder nachgeregelt werden muss. In der Regel geschieht dies durch Anreicherung der Raumluft mit Inertgas (aus Flaschenbatterien oder Generatoren, insb. Stickstoff, bei Bedarf auch Argon), aber in manchen Anlagen auch durch O2-Abreicherung der Raumluft und Wiedereinleitung dieser abgereicherten Luft.

Es gibt mehrere Regelungs-Varianten solcher Systeme. Bspw. kann der O2-Gehalt bei 17% gehalten werden, was noch Personenverkehr ohne Atemschutz ermöglicht, wobei bei z.B. Rauchgasdetektion der Bereich evakuiert wird und der O2-Gehalt auf 15%, 13% oder 11% gesenkt wird um den Brand zu ersticken. Alternativ kann der O2-Gehalt auch nochmals gesenkt werden, wenn auch nach vorgegebener Zeit Rauchgas detektiert wird. Alternativ kann der O2-Gehalt auch dauerhaft unterhalb der Entzündungskonzentration, bspw. bei 13% gehalten werden.

Die zuverlässige und schnelle Erfassung des O2-Gehalts ist also essentiell für solche Anlagen. Hierfür werden Sauerstoffdetektoren verwendet, welche beispielsweise eine elektrochemische Zelle (EC) verwenden, die dauerhaft geheizt werden muss (500 mA Heizstrom), und über einen 4-20 mA Ausgang verfügt, d.h. der gemessene O2-Gehalt zwischen zwei vorbestimmten Grenzwerten wird im Melder in ein analoges Stromsignal übersetzt: 4 mA Betriebsstrom + Strombeitrag 0-16 mA, 0 mA für unteren und 16 mA für oberen O2%-Grenzwert. Das 4-20 mA Signal eines einzelnen Teilnehmers wird über eine Zweidrahtleitung direkt an die Zentrale übertragen.

Der 500 mA Heizstrom erfordert einen dritten Draht (Ground von Zweidrahtleitung, oder eigenen vierten Draht als Ground), was die Verkabelung auch aufgrund der hohen Stromlast des dritten Drahts (zusätzliche Isolierung) sehr aufwändig macht. Der dritte Draht kann auch von einer anderen, nur stromversorgenden Stelle kommen als die Meldezentrale.

Vor diesem Hintergrund lag der Erfindung die Aufgabe zugrunde, einen Gasdetektor bereitzustellen, der zumindest eines der oben aufgezeigten Probleme löst oder die oben genannten Anforderungen besonders gut erfüllt.

Gemäß einem ersten Aspekt der Erfindung wird diese Aufgabe durch einen Gasdetektor gelöst, der aufweist: einen elektrochemischen Sensor, EC-Sensor, der zur Detektion einer Konzentration wenigstens eines Gases oder Gasgemischs ausgebildet ist, wobei der EC-Sensor mehrere Elektroden, die auf einer Seite des EC-Sensors ausgebildet sind, und einen Gaseingang aufweist, eine Sensorplatine, die in Verbindung mit den mehreren Elektroden des EC-Sensors steht, und ein Detektorgehäuse, das den EC-Sensor und die Sensorplatine umschließt, wobei das Detektorgehäuse einen Gaseinlass mit einer Einlassöffnung aufweist, wobei das Detektorgehäuse ein Gasausgleichsvolumen im Inneren zwischen Detektorgehäuse und EC-Sensor definiert, das eine Gasströmung zu dem Gaseingang des EC-Sensors ermöglicht.

Ein EC-Sensor ausgebildet zur Detektion einer Konzentration wenigstens eines Gases oder Gasgemischs macht sich die chemischen Eigenschaften des Gases oder des Gasgemischs, insbesondere Reaktivität, Oxidierbarkeit und Reduzierbarkeit zunutze, erhält per Messung eine chemische Information über das Gas oder das Gasgemisch und setzt diese chemische Information in eine elektronische Information um. Diese Umsetzung geschieht beispielsweise resistiv, wobei das zu messende Gas oder Gasgemisch direkt die Leitfähigkeit einer gasempfindlichen Sensorschicht beeinflusst (beispielsweise durch einen Chemo-Resistor wie einen anorganischen Metalloxid-Halbleiter, ein organisches Phthalocyanin oder ein leitfähiges Polymer), kapazitiv, wobei hier die Kapazität eines Kondensators durch ein gasempfindliches Dielektrikum beeinflusst wird (beispielsweise Polymersensoren), potentiometrisch, wobei vom Sensor selbst eine Spannung erzeugt wird, die direkt messbar ist (beispielsweise ein Festkörper-lonenleiter oder ein Chemo-Transistor), amperometrisch, wobei ein messbarer Strom vom amperometrischen Sensor geliefert wird (beispielsweise eine elektrochemische Zelle, ein Flammenionisationsdetektor oder ein Photoionisationsdetektor), thermisch, wobei die Temperaturerhöhung aufgrund einer chemischen Reaktion an der Sensoroberfläche gemessen oder direkt die Wärmeleitfähigkeit des Gases als Messgröße verwendet wird, thermochemisch, wobei auf der Sensoroberfläche chemische Reaktionen stattfinden, bei denen Energie in Form von Wärme abgegeben wird, was zu einer messbaren Temperaturerhöhung führt (beispielsweise ein Wärmetönungssensor wie ein Pellistor), thermisch-physikalisch, wobei eine direkte Messung der Wärmeleitfähigkeit der Gasatmosphäre durchgeführt wird (beispielsweise ein Wärmeleitfähigkeitsdetektor), gravimetrisch, wobei eine Massenänderung gemessen wird, hervorgerufen durch Gasmoleküle, die sich beispielsweise auf der Oberfläche eines Schwingquarzes ablagern und dadurch seine Resonanzfrequenz verändern (beispielsweise Sensoren, die nach dem Prinzip eines piezoelektrischen Sensors arbeiten wie Quarzkristall-Mikrowaagen oder Sensoren, die akustische Oberflächenwellen messen), optisch, wobei optische Eigenschaften eines mit Gas gefüllten Probenraumes genutzt werden (beispielsweise anhand der Messung des Brechungsindex, Absorptionsspektrum, Intensität oder Lumineszenz) oder biochemisch, die dem biologischen Vorbild der Umsetzung von bestimmten Stoffen oder Stoffgruppen folgen (beispielsweise Biosensoren).

Ein EC-Sensor gemäß der vorliegenden Erfindung ist bevorzugt dazu ausgebildet eine Sauerstoffkonzentration, eine Konzentration eines Sauerstoffgemisches oder eine Konzentration eines oder mehrere weiterer Gase zu detektieren, die bei einer chemischen Reaktion umfassend Sauerstoff als Edukte oder Produkte vorkommen.

Ein erfindungsgemäßer EC-Sensor weist mehrere Elektroden, die auf einer Seite des EC-Sensors ausgebildet sind, und einen Gaseingang auf. Die mehreren Elektroden dienen zur Bereitstellung eines Stromkreises mit der Sensorplatine zur Übertragung elektronischer Informationen, beispielsweise zur Übertragung von Messdaten insbesondere einer elektrischen Spannung. Insbesondere weist der EC-Sensor drei Elektroden auf, eine Referenzelektrode (Reference Electrode), eine Messelektrode (Sensing Electrode) und eine Gegenelektrode (Counter Elektrode). Der EC-Sensor funktioniert beispielsweise derart, indem er auf das zu detektierende Gas oder Gasgemisch reagiert und dann ein elektrisches Ausgangssignal als elektronische Information erzeugt, das proportional zur Gaskonzentration ist. Das zu detektierende Gas oder Gasgemisch, das mit dem Sensor in Berührung kommt, tritt zunächst durch den Gaseingang und erreicht die Oberfläche der Messelektrode. Die Moleküle auf der Elektrode werden sofort oxidiert oder reduziert, um Elektronen zu erzeugen oder zu verbrauchen und einen elektrischen Strom zu erzeugen. Hierbei handelt es sich bevorzugt um einen katalysierten Prozess, der auf den speziell für das zu detektierende Gas oder Gasgemisch entwickelten Elektrodenmaterialien beruht. Alternativ sind EC-Sensoren mit zwei Elektroden bekannt, bei denen auf die Referenzelektrode verzichtet wird. Grundsätzlich sind erfindungsgemäß sämtliche bekannte EC-Sensoren zur Gasdetektion vorteilhaft einsetzbar.

Die Sensorplatine, die in Verbindung mit den mehreren Elektroden des EC-Sensors steht, dient insbesondere zur Übermittlung von Steuerinformationen an den EC-Sensor und Übermittlung von Messdaten von dem EC-Sensor, insbesondere einer gemessenen elektrischen Spannung.

Das Detektorgehäuse umschließt den EC-Sensor und die Sensorplatine, wobei das Detektorgehäuse einen Gaseinlass mit einer Einlassöffnung aufweist und das Detektorgehäuse ein Gasausgleichsvolumen im Inneren zwischen Detektorgehäuse und EC-Sensor definiert, das eine Gasströmung zu dem Gaseingang des EC-Sensors ermöglicht.

Erfindungsgemäß weist das Detektorgehäuse explizit nur einen Gaseinlass und nicht mehrere örtlich getrennte Öffnungen des auf, da erkannt wurde, dass dies einen verbesserten Gasstrom und damit Gasausgleich des Gasausgleichsvolumens in den Gasdetektor ermöglicht, was eine schnellere Detektion einer Konzentrationsänderung ermöglicht.

Gemäß einer vorteilhaften Ausgestaltung des ersten Aspekts der Erfindung, weist der EC-Sensor einen Gasauslass auf der dem Gaseingang gegenüberliegenden Seite auf, wobei eine Gasströmung ausgehend von dem Gaseinlass des Detektorgehäuses durch den EC-Sensor von dem Gaseingang zu dem Gasauslass und zurück zu dem Gaseinlass des Detektorgehäuses in einem Luftspalt zwischen Detektorgehäuse und EC-Sensor ausgebildet ist.

Hierdurch wird eine bessere Gasströmung im Gasdetektor und dadurch ein besserer Gasaustausch ermöglicht, was zu einer verbesserten Messgenauigkeit und einer schnelleren Detektion führt. Durch den verbesserten Gasaustausch gelangt das Gas oder das Gasgemisch aus dem von dem Gasdetektor zu überwachenden Raum besser und schneller an den EC-Sensor. Somit wird eine Änderung einer Konzentration des Gases oder des Gasgemisches schneller detektiert und Maßnahmen der aktiven Brandvermeidung können schneller eingeleitet werden.

Gemäß einer weiteren vorteilhaften Ausgestaltung des ersten Aspekts der Erfindung, weist der EC-Sensor eine im Wesentlichen zylindrische Form auf, wobei die Elektroden und der Gaseingang des EC-Sensor auf jeweils gegenüberliegenden Stirnflächen der zylindrischen Form ausgebildet sind.

Hierdurch wird der Gasstrom in den Gaseingang nicht durch die Elektroden beeinträchtigt, was zu einem verbesserten Gasaustausch und somit einer schnelleren Detektion einer Konzentrationsänderung führt.

Insbesondere sind die Elektroden auf einer ersten Stirnseite des zylindrischen EC-Sensors ausgebildet und der Gaseingang auf einer der ersten Stirnseite gegenüberliegenden zweiten Stirnseite ausgebildet.

In einer bevorzugten Variante der obigen Ausgestaltung definiert das Detektorgehäuse einen axial entlang des Zylinders verlaufenden Luftspalt radial außerhalb zwischen Zylinder des EC-Sensors und Detektorgehäuse.

Dieser Luftspalt verbessert den Gasstrom durch und um den Zylinder was zu einem besseren Gasaustausch und einer schnelleren Detektion einer Konzentrationsänderung führt.

Die Formulierung "axial entlang des Zylinder" bezieht sich auf eine Richtung einer Symmetrieachse des Zylinders, insbesondere einer Symmetrieachse, die durch den Mittelpunkt einer ersten Stirnseite des Zylinders und einer zweiten Stirnseite des Zylinders führt.

Die Formulierung "radial außerhalb des Zylinders" bezieht sich auf eine Richtung eines Radius einer ersten Stirnseite des Zylinders und einer zweiten Stirnseite des Zylinders ausgehend von deren Mittelpunkt radial nach außen.

Der Luftspalt ist also in einer Richtung parallel zu einer Symmetrieachse des Zylinders zwischen einer Außenwand des Zylinders und einer Innenwand des Detektorgehäuses ausgebildet.

Gemäß einer weiteren vorteilhaften Ausgestaltung des ersten Aspekts der Erfindung, weist der Gasdetektor weiter einen Einleger auf, der zwischen EC-Sensor und Sensorplatine angeordnet ist, derart, dass das von Luft eingenommene Volumen zwischen EC-Sensor und Sensorplatine, nämlich das Gasausgleichsvolumen, verkleinert ist, wobei insbesondere der Abstand zwischen EC-Sensor und Sensorplatine durch die Elektroden definiert ist.

Der Einleger weist insbesondere mehrere Öffnungen auf, ausgestaltet zur Aufnahme der mehreren Elektroden des Gasdetektors, wobei bevorzugt jede Öffnung dazu ausgestaltet ist eine Elektrode aufzunehmen.

Durch die Verringerung des von Luft eingenommenen Volumens zwischen EC-Sensor und Sensorplatine wird ein besserer und vor allem schnellerer Gasaustausch innerhalb des EC-Sensors ermöglicht, was zu einer schnelleren Detektion einer Konzentrationsänderung führt.

Gemäß einer weiteren vorteilhaften Ausgestaltung des ersten Aspekts der Erfindung, weist der Gasdetektor weiter eine Gasdetektorplatine auf, wobei die Gasdetektorplatine innerhalb des Detektorgehäuse und in Kommunikationsverbindung mit der Sensorplatine angeordnet ist, wobei die Gasdetektorplatine dazu ausgebildet ist, ein Gasdetektionssignal von der Sensorplatine zu empfangen und in Abhängigkeit des Gasdetektionssignals mit einer weiteren Einheit, insbesondere einer Brandmelder- und/oder Löschsteuerzentrale, zu kommunizieren.

Insbesondere ist die Gasdetektorplatine dazu ausgestaltet, abhängig von dem Gasdetektionssignal der Sensorplatine, beispielsweise bei Übersteigung eines Schwellenwertes für das Gasdetektionssignal, ein Einleiten von Mitteln zur aktiven Brandvermeidung durch die Kommunikation mit der weiteren Einheit herbeizuführen.

Gemäß einer weiteren vorteilhaften Ausgestaltung des ersten Aspekts der Erfindung, weist das Detektorgehäuse einen Gehäusesockel, der insbesondere an einer Wand verschraubbar ausgestaltet ist, und einen darauf montierbaren, insbesondere daran verschraubbaren, Gehäuseabschnitt auf.

Dieser Gehäusesockel weist insbesondere Aufnahmen für Befestigungsmittel, insbesondere Schrauben, auf, mithilfe derer das Detektorgehäuse, insbesondere an einer Wand, befestigt werden kann. Überdies weist der Gehäusesockel insbesondere weitere Aufnahmen für Befestigungsmittel, insbesondere Schrauben, auf, mithilfe derer der montierbare Gehäuseabschnitt an dem Gehäusesockel befestigt werden kann.

Der montierbare Gehäuseabschnitt ist bevorzugt zur Aufnahme des EC-Sensors sowie der Sensorplatine und insbesondere auch der Gasdetektorplatine ausgestaltet und weist besonders bevorzugt weitere Aufnahmen zur Aufnahme von Befestigungsmitteln, insbesondere Schrauben, auf, mithilfe derer der montierbare Gehäuseabschnitt an dem Gehäusesockel befestigt wird.

Gemäß einer bevorzugten Variante der obigen Ausgestaltung weist der Gehäuseabschnitt eine Aufnahme für ein Belüftungselement mit dem Gaseinlass des Detektorgehäuses an einer dem Gehäusesockel gegenüberliegenden Seite, insbesondere mittig an der dem Gehäusesockel gegenüberliegenden Seite, auf.

Eine mittige Anordnung der Aufnahme für ein Belüftungselement ermöglicht eine besonders kompakte Ausgestaltung des Gasdetektors.

Gemäß einer besonders bevorzugten Variante der obigen Ausgestaltung und der obigen Variante, definiert das Detektorgehäuse mit Ausnahme der Aufnahme für das Belüftungselement einen dichten Aufnahmeraum für den EC-Sensor.

Dicht bedeutet in diesem Kontext, dass abgesehen durch die Aufnahme für das Belüftungselement kein Gas in den Aufnahmeraum für den EC-Sensor von außerhalb des Gasdetektors eindringen kann. Insbesondere ist der Aufnahmeraum luftdicht ausgestaltet mit Ausnahme der Aufnahme für das Belüftungselement.

Dies führt zu einem verbesserten Gasstrom und Gasaustausch in dem Gasdetektor, was eine schnellere Detektion einer Konzentrationsänderung ermöglicht.

Der Gehäuseabschnitt ist überdies insbesondere konusförmig ausgestaltet, was eine besonders kompakte Ausgestaltung des Gasdetektors ermöglicht.

Gemäß einer weiteren vorteilhaften Ausgestaltung des ersten Aspekts der Erfindung, stellt der EC-Sensor ein analoges Stromsignal zwischen zwei der Elektroden als Detektionssignal, insbesondere eine Spannung im nV-Bereich, bereit.

Dieses Detektionssignal resultiert aus der Detektion des Gases oder des Gasgemisches. Die Spannung im Nanovolt-Bereich wird durch eine chemische Reaktion des Gases oder des Gasgemisches an den Elektroden erzeugt.

Dieses Detektionssignal wird vom Gasdetektor, insbesondere von der Sensorplatine, in einen Strom als elektronische Information, insbesondere mit einer Stromstärke von 4 bis 20 mA umgewandelt, die wiederum der Gasdetektorplatine bereitgestellt wird. Die Gasdetektorplatine führt anhand dieser Stromstärke, beispielsweise bei Übersteigung eines Schwellenwertes für das Detektionssignal, ein Einleiten von Mitteln zur aktiven Brandvermeidung durch die Kommunikation mit der weiteren Einheit herbei.

Hierbei variiert die Stromstärke basierend auf dem Detektionssignal des EC-Sensors insbesondere abhängig von der O2-Konzentration und setzt sich bevorzugt wie folgt zusammen: 4 mA Betriebsstrom + Strombeitrag 0-16 mA, 0 mA für einen unteren Grenzwert für die O2-Konzentration und 16 mA für einen oberen Schwellenwert der O2-Konzentration. Bei einer Stromstärke von beispielsweise 20 mA führt die Gasdetektorplatine dann ein Einleiten von Mitteln zur aktiven Brandvermeidung durch die Kommunikation mit der weiteren Einheit herbei.

Gemäß einer weiteren vorteilhaften Ausgestaltung des ersten Aspekts der Erfindung, weist der Gaseinlass eine Gas-permeable Membran auf.

Bei dem Durchdringen der Gas-permeable Membran diffundiert das Gas, was eine gleichmäßigere Konzentration des zu detektierenden Gases oder des Gasgemisches im Gasdetektor herbeiführt.

Dies ermöglicht eine genauere Detektion der tatsächlichen Konzentration des zu detektierenden Gases oder Gasgemisches in dem einströmenden Gas, da lokale Konzentrationsmaxima durch die Diffusion an der Gas-permeablen Membran ausgeglichen werden.

Gemäß einer weiteren vorteilhaften Ausgestaltung des ersten Aspekts der Erfindung, ist der Gasdetektor nicht zur Beheizung des EC-Sensors ausgebildet.

Dies ermöglicht eine kompaktere Ausgestaltung des Gasdetektors, da der Gasdetektor ohne Verkabelung des EC-Sensors zur Übertragung eines Heizstroms und Isolierung dieser Verkabelung, notwendig durch die relativ hohe Stromlast des Heizstroms gegenüber der Stromlast des Detektionssignals, ausgestaltet werden kann.

Gemäß einer weiteren vorteilhaften Ausgestaltung des ersten Aspekts der Erfindung, weist der Gasdetektor einen Temperatursensor auf und korrigiert ein Detektionswert des EC-Sensors mittels eines von dem Temperatursensor erfassten Wertes korrigiert.

Somit ist es möglich die Auswirkungen thermisch bedingter Änderungen der Eigenschaften des Gases oder des Gasgemisches auf den Detektionswert zu korrigieren und die Genauigkeit des Messergebnisses zu erhöhen.

Der Temperatursensor ist dabei insbesondere mit der Sensorplatine kommunikativ verbindbar ausgestaltet, sodass der Temperatursensor Messdaten an die Sensorplatine übermitteln kann. Die Sensorplatine führt dann anhand der von dem EC-Sensor erhaltenen elektronischen Information über die gemessene Konzentration und der Messdaten des Temperatursensors eine Korrektur der elektronischen Information durch und ermittelt einen korrigierten Wert für die gemessene Konzentration.

Gemäß einer weiteren vorteilhaften Ausgestaltung des ersten Aspekts der Erfindung, ist der EC-Sensor in dem Detektorgehäuse mit mehreren Dichtungen eingefasst.

Dies ermöglicht eine dichte Fixierung des EC-Sensors in dem Detektorgehäuse.

Gemäß einer bevorzugten Variante der obigen Ausgestaltung weisen die mehreren Dichtungen eine zylindrische Flachdichtung, die an der Sensorplatine radial nach außen abdichten, und/oder einen O-Ring zwischen Sensorplatine bzw. Flachdichtung und Gaseinlass, und/oder eine zylindrische Dichtung mit seitlicher Unterbrechung am vorderen Ende des EC-Sensors einen Gaseingang des EC-Sensors umschließend auf.

Insbesondere wenn der Gasdetektor einen Gasauslass aufweist sind die zylindrische Flachdichtung und die zylindrische Dichtung besonders vorteilhaft, da diese eine Trennung des Gasstroms in den EC-Sensor und den Gasstrom aus dem EC-Sensor voneinander trennen. Dies führt zu einer verbesserten Gasströmungsdynamik im Gasdetektor und ermöglicht eine genauere Detektion der Konzentration und eine schnellere Detektion einer Konzentrationsänderung.

Durch die seitliche Unterbrechung am vorderen Ende des EC-Sensors der zylindrische Dichtung kann insbesondere das Gas aus dem Gasauslass des EC-Sensors aus dem Gasdetektor geleitet werden.

Gemäß einer weiteren vorteilhaften Ausgestaltung des ersten Aspekts der Erfindung, ist ein Gasauslass des EC-Sensors im Druckequilibrium mit dem Gaseingang des EC-Sensors.

Dies verhindert eine druckbedingte Verschlechterung der Gasströmungsdynamik durch den EC-Sensor und ermöglich so eine verbesserte Detektion.

Gemäß einer weiteren vorteilhaften Ausgestaltung des ersten Aspekts der Erfindung, beträgt das Gasausgleichsvolumenzwischen Detektorgehäuses und EC-Sensor höchstens 2.000 mm³, vorzugsweise höchstens 1.700 mm³ und besonders bevorzugt höchstens etwa 1.200 mm³.

Dies ermöglicht eine verbesserte Gasströmungsdynamik im Gasdetektor, was eine genauere Messung der Konzentration und schnellere Detektion eine Konzentrationsänderung ermöglicht.

Insbesondere eine Ausgestaltung des Gasdetektors mit einer Einlage ist besonders vorteilhaft, da das freie Gasvolumen zwischen Detektorgehäuses und EC-Sensor in dieser Ausgestaltung lediglich etwa 1.200 mm³ beträgt.

Gemäß einer weiteren vorteilhaften Ausgestaltung des ersten Aspekts der Erfindung, ist der Gasdetektor über genau zwei Anschlussleitungen mittels einer Grenzwertlinie und/oder einem Loop-Ring und/oder eine 4-20mA Zweidrahtleitung mit einer Brandmelder- und/oder Löschsteuerzentrale verbindbar.

In einem weiteren Aspekt wird ein Verfahren zur Detektion einer Gaskonzentration in einem Gasdetektor vorgeschlagen, umfassend: Bereitstellen eines elektrochemischen Sensors, EC-Sensor, der zur Detektion einer wenigstens eines Gases oder Gasgemischs ausgebildet ist, wobei der EC-Sensor mehrere Elektroden, die auf einer Seite des EC-Sensors ausgebildet sind, und einen Gaseingang aufweist; Verbinden einer Sensorplatine mit den mehreren Elektroden des EC-Sensors, und Umschließen des EC-Sensors und der Sensorplatine mit einem Detektorgehäuse, wobei das Detektorgehäuse bis auf einen Gaseinlass mit einer Einlassöffnung geschlossen ist, wobei das Detektorgehäuse ein Gasausgleichsvolumen im Inneren zwischen Detektorgehäuse und EC-Sensor definiert, das eine hinreichende Gasströmung zu dem Gaseingang des EC-Sensors ermöglicht.

Die Gasströmung ist insbesondere dann hinreichend, wenn eine sich in der Umgebung einstellende Änderung einer Gaskonzentration innerhalb eines definierten Zeitraums an dem EC-Sensor einstellt. Hierfür muss das geschützte Volumen, das Gasausgleichsvolumen, innerhalb des Gasdetektors und außerhalb des EC-Sensors hinreichend klein sein. Hinreichend ist hier für einen Fachmann (m/w/d) anhand der Spezifikationen des Gasdetektors festzulegen, wobei grundsätzlich gilt, dass ein kleineres Gasausgleichsvolumen eine schnellere Einregelung auf die geänderte Gaskonzentration ermöglicht. Zudem muss das Gasausgleichsvolumen einen ungestörten Zugang des Gases zu allen Ein- und Auslässen des EC-Sensors ermöglichen, was einer beliebigen Verkleinerung des Gasausgleichsvolumens entgegensteht.Im Folgenden wird die vorliegende Erfindung anhand von in den Figuren dargestellten Ausführungsbeispielen weiter illustriert und erläutert. Hierbei zeigt
- Fig. 1: eine schematische Darstellung zur Illustration eines Ausführungsbeispiels eines Gasdetektors,
- Fig. 2: eine schematische Darstellung zur Illustration eines Ausführungsbeispiels des Gasdetektors in einer Explosionszeichnung,
- Fig. 3: mehrere Ansichten eines beispielhaften EC-Sensors,
- Fig. 4A-4C: Schnittansichten und Detailansichten des Gasdetektors,
- Fig. 5: eine schematische Darstellung zur Illustration der in Fig. 4C gezeigten Detailansicht des Gasdetektors in einer Explosionszeichnung,
- Fig. 6: ein Diagramm zur Veranschaulichung der Bestimmung einer T90 Zeit
- Fig. 7: ein Diagramm der detektierten O2-Konzentration von verschiedenen Gasdetektoren über die Zeit

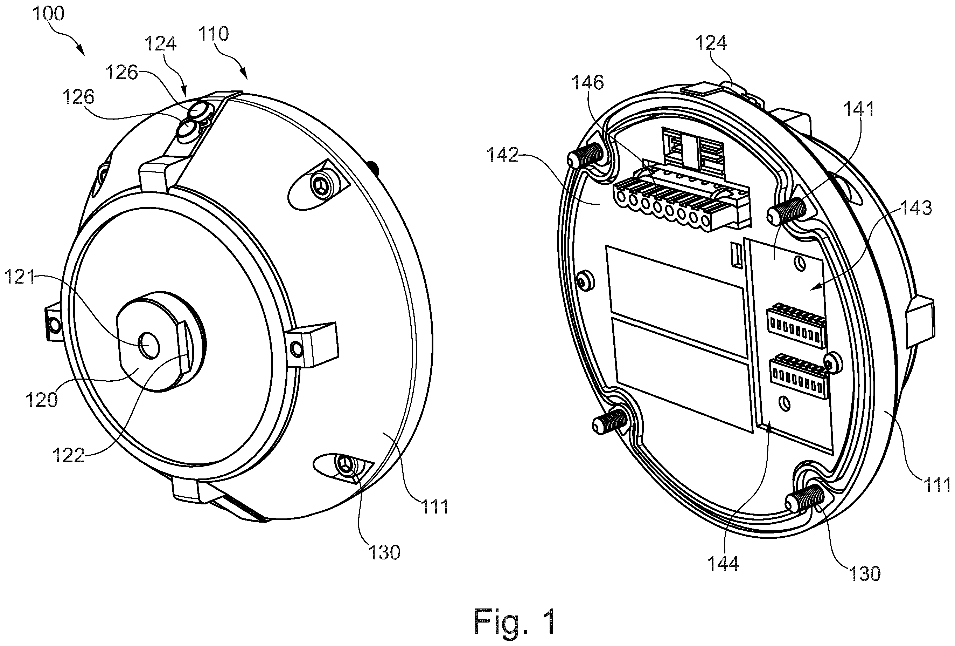

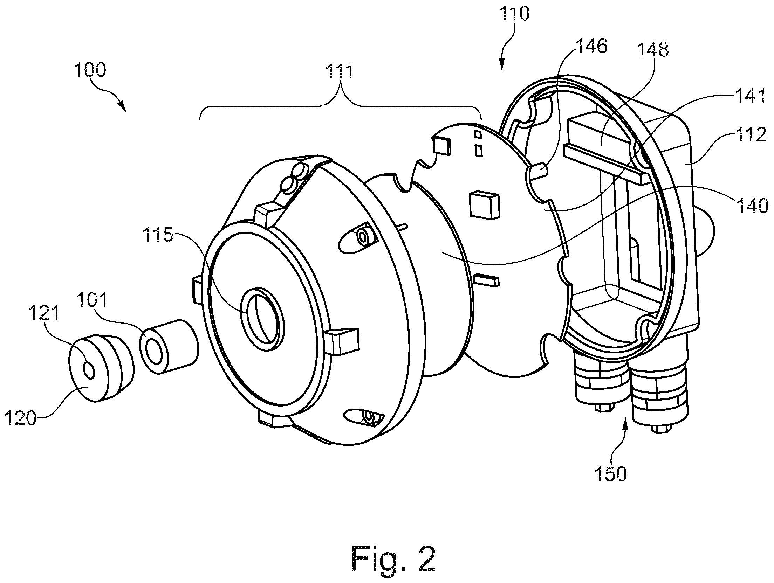

Die Figur 1 zeigt einen Gasdetektor 100 in Form einer Melder-Plattform mit einem mittigen Gaseinlass 120 an der Oberfläche des Gehäuseabschnittes 111. Das Detektorgehäuse 110 besteht aus Gehäuseabschnitt 111 und Gehäusesockel 112, der in Fig. 2 gezeigt ist. Der Gehäuseabschnitt 111 weist Befestigungsmittel 130 in Form von Schrauben in passenden Aufnahmen des Gehäusesockels 112 auf.

In Fig. 1 ist links eine perspektivische Ansicht von vorne und rechts eine perspektivische Ansicht von hinten auf den Gehäuseabschnitt 111 des Gasdetektors 100 zu sehen.

Im Zentrum des Gaseinlasses 120 ist eine Gas-permeable Membran 121 angeordnet, die vermeidet, das Staub und Verschmutzung auf den dahinterliegenden (in Fig. 1 nicht gezeigten EC-Sensor 101 treffen können.

Der Gaseinlass 120 ist insbesondere an der Vorderseite des Gehäuseabschnittes 111 in diesen einschraubbar und weist hierfür ein geeignetes Antriebsprofil 122 auf. Beispielsweise ist das Antriebsprofil 122 als 2-Kant Profil mit zwei gegenüberliegenden Backen ausgeführt. Mittels des Antriebsprofils 122 kann der Gaseinlass 120 demnach beispielsweise bei Verschmutzung ausgetauscht werden, ohne dass das gesamte Detektorgehäuse 110 zu öffnen ist.

Der Gehäuseabschnitt 111 weist ferner einen Anzeigebereich 124, der in diesem Beispiel zwei LED Anzeigen umfasst, auf. Auf diesem kann beispielsweise ein Status des Gasdetektors 100 optisch angezeigt werden. Auch andere Arten der Statuskommunikation, beispielsweise akustisch oder per Signalübertragung an eine Zentrale, sind möglich.

Den rückseitigen Bereich des Gehäuseabschnittes 111 deckt überwiegend eine Abdeckplatte 142 ab. Die Abdeckplatte 142 schützt insbesondere eine dahinterliegende Gasdetektorplatine 141. Eine Aussparung 144 ermöglicht einen direkten Zugriff auf einen Teil der Gasdetektorplatine 141, nämlich einen Bereich mit einem Konfigurationsmittel 143, beispielsweise in Form von DIP-Schaltern. Über das Konfigurationsmittel 143 können Einstellungen, beispielsweise Adressierung und/oder Meldungseigenschaften, des Gasdetektors 100 vorgenommen werden.

Ein Konnektor 146, der beispielsweise als 8 Pin Konnektor ausgeführt ist, steht vor der Abdeckplatte 142 hervor. Der Konnektor 146 ist ausgebildet, auf einen entsprechenden Konnektor 148 des Gehäusesockels 112 (vgl. Fig. 2) aufgesteckt zu werden. Mittels der beiden Konnektoren 146, 148 wird die Funktionalität des Gasdetektors 100, die in dem Gehäuseabschnitt 111 steckt, an eine Melderleitung angeschlossen, die in den Gehäusesockel 112 verläuft.

Figur 2 zeigt eine Explosionszeichnung eines erfindungsgemäßen Gasdetektors 100. Der Gasdetektor 100 umfasst den Gehäuseabschnitt 111, einen Gehäusesockel 112, den Gaseinlass 120, einen EC-Sensor 101, eine Sensorplatine 140 und die Gasdetektorplatine 141. Die weiteren aus Fig. 1 bekannten Elemente sind vorzugsweise ebenfalls enthalten und zur Verbesserung der Darstellung in der Figur nicht ausdrücklich benannt.

Der EC-Sensor 101 ist beispielsweise ein O2-Gassensor und wird gemäß einer auf dem Gehäuseabschnitt 111 mittig angeordnete Aufnahme 115 in dem Detektorgehäuse 110 angeordnet, sodass der Gaseinlass 110 von der mittigen Aufnahme 115 aufgenommen wird und mit dem EC-Sensor 101 in direktem Kontakt steht. Der Gaseingang des EC-Sensors 101 ist geschützt durch den Gaseinlass 120, welcher eine Gas-permeable Membran 121 umfasst und ansonsten dicht ist, um den EC-Sensor 101 zu schützen. Der EC-Sensor 101 ist aufgesteckt auf die Sensorplatine 140, welche wiederum mit der Gasdetektorplatine 141 verbunden ist.

Der Gehäusesockel 112 wird insbesondere an einer Wand verschraubt und weist den beschriebenen Konnektor 148 und eine bzw. mehrere Kabeleinführungen 150 zum Einführen von Melderleitungen auf. Die Melderleitungen werden dann mit dem Konnektor 148 verbunden. Der Konnektor 148 bietet vorzugsweise die Möglichkeit, ein KNX Gateway oder andere Schnittstellen zur Kommunikation über die Melderleitungen zwischen Melderleitungen und dem Konnektor 148 anzuschließen. Der Gehäusesockel 112 weist eine Schnittstelle auf, die mit einer korrespondierenden Schnittstelle des Gehäuseabschnittes 111 bündig abschließt, so dass die beiden Teile des Detektorgehäuses 110 zuverlässig miteinander verbindbar sind.

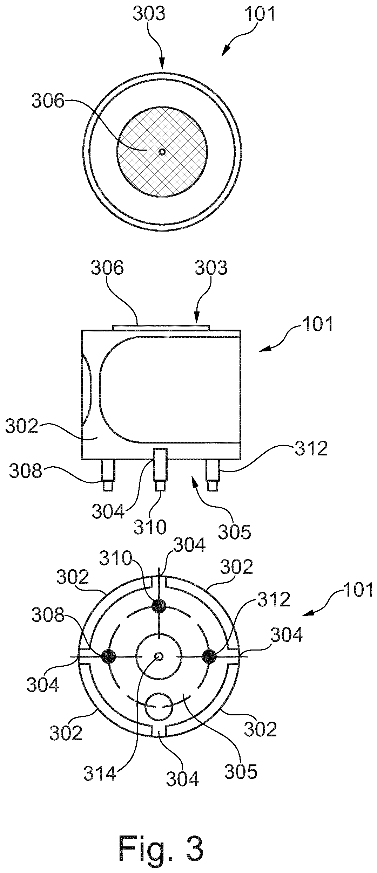

Fig. 3 zeigt schematisch und exemplarisch drei Ansichten auf einen beispielhaften EC-Sensor 101. Der EC-Sensor 101 ist im Wesentlichen zylinderförmig mit einer umlaufenden Mantelfläche 302. über der oberen Stirnfläche 303 steht eine gaspermeable Staubabdeckung 306 hervor. Die Staubabdeckung 306 bedeckt einen mittleren Bereich der Stirnfläche 303, unter der sich die eigentlichen Sensorelemente befinden. Die Staubabdeckung 306 ist gleichzeitig der Gaseinlass des EC-Sensors 101.

Von der unteren Stirnfläche 305, die der oberen Stirnfläche 303 gegenüberliegt, stehen drei Elektroden 308, 310, 312 hervor, nämlich die Referenzelektrode 308 (*reference electrode*), die Arbeitselektrode 310 (*working electrode*) und die Gegenelektrode 312 (*counter electrode*).

Die Mantelfläche 302 erstreckt sich über die untere Stirnfläche 305 hinaus nach unten, wobei hierbei Unterbrechungen 304 in der Mantelfläche 302 ausgebildet sind. Somit ist der hervorstehende Kragenabschnitt der Mantelfläche 302 nicht durchgehend sondern unterbrochen ausgeführt.

In der Mitte der unteren Stirnfläche 305 ist ferner eine Lüftungsöffnung 314 angeordnet. In anderen Ausführungen des EC-Sensors 101 ist die Referenzelektrode 308 und/oder die Lüftungsöffnung 314 nicht vorgesehen.

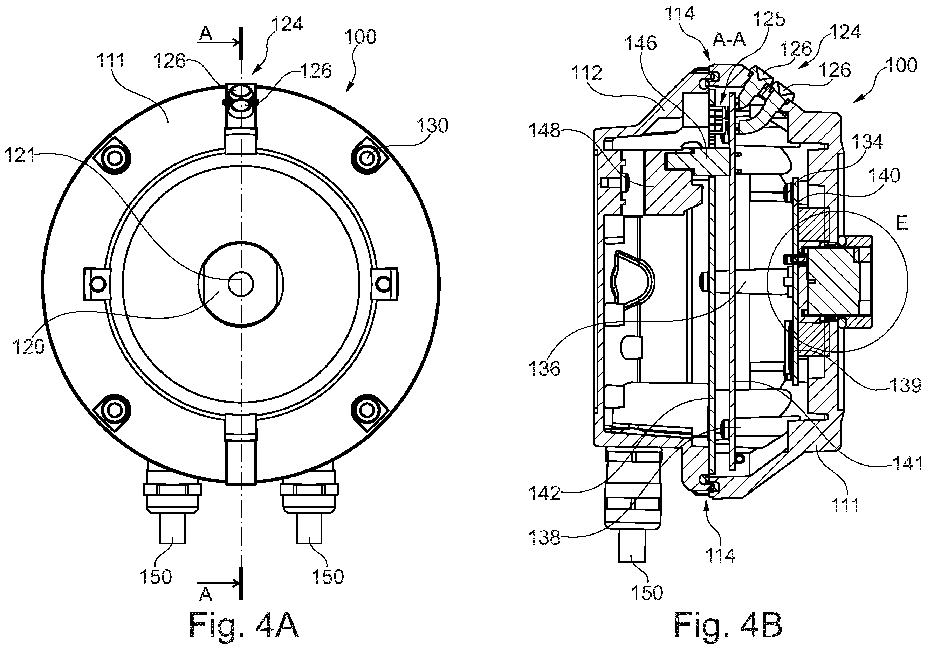

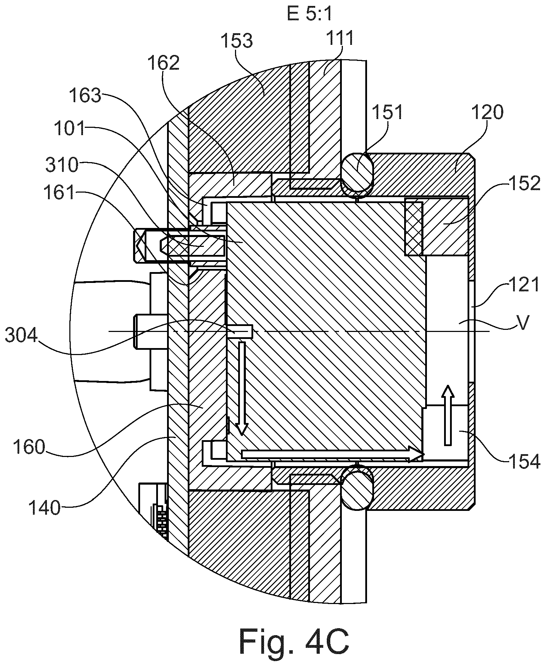

Fig. 4 zeigt einen Querschnitt durch die Mitte des Gasdetektors 100, wobei in Fig. 4A die Schnittachse A-A in einer Draufsicht auf den Gasdetektor 100 eingezeichnet ist, Fig. 4B einen Querschnitt durch den Gasdetektor 100 entlang der Schnittachse A-A zeigt und Fig. 4C eine Vergrößerung des in Fig. 4B eingezeichneten Abschnittes E darstellt.

In Fig. 4B ist die Verbindung von Gehäuseabschnitt 111 und Gehäusesockel 112 sichtbar. Umlaufend ist eine in Art einer Nut-Feder-Verbindung ausgeführte Dichtverbindung 114 zu sehen, die eine zuverlässige Dichtung gewährleistet.

Zur Ansteuerung der LED 126 ist im Anzeigebereich 124 eine LED Steuerelektronik 125 ausgebildet.

Die Sensorplatine 140 ist mittels eines Befestigungsmittels 134, die Gasdetektorplatine 141 mittels eines Befestigungsmittels 136 und die Abdeckplatte 142 mittels eines Befestigungsmittels 138 mit dem Gehäuseabschnitt 111 verbunden bzw. daran befestigt. Auf der Sensorplatine 140 ist eine Umwandlungselektronik 139 zur Umwandlung der Ausgangssignale des EC-Sensors 101 in ein für die Gasdetektorplatine 141 verwendbares Messsignal vorgesehen.

Für die Erfindung zentrale Komponenten sind in Fig. 4C vergrößert dargestellt, diese sind zur besseren Lesbarkeit in Fig. 4B nicht bezeichnet.

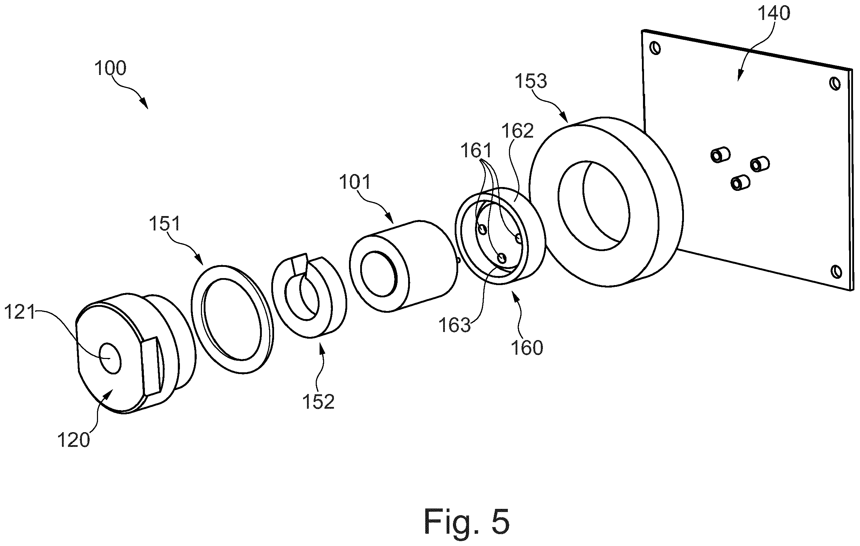

Figur 5 zeigt eine Explosionszeichnung der in der vergrößerten Darstellung der Fig. 4C gezeigten Komponenten des Gasdetektors 100.

Zwischen Gaseinlass 120 mit Membran 121 und Sensorplatine 140 ist der EC-Sensor 101 in einer Reihe an Dichtungen 151, 152, 153 eingefasst. Die als O-Ring ausgeführte Dichtung 151 dichtet den Gaseinlass 120 gegen den Gehäuseabschnitt 111 ab.

Die Dichtung 152 dichtet den EC-Sensor 101 in axialer Richtung gegen den Gaseinlass 120 ab. Zudem kann sie aufgrund der vorstehenden Staubabdeckung 306 auch eine Zentrierwirkung in radialer Richtung ausüben. Sie ist im Wesentlichen zylindrisch ausgeführt, weist aber eine Unterbrechung 154 von insbesondere zwischen 5° und 90° in Umfangsrichtung auf. Die Unterbrechung 154 ermöglicht einen Gasstrom über die Dichtung 152 in axialer Richtung der Dichtung 152, nämlich genau im Bereich der Unterbrechung 154 wie durch einen Pfeil schematisch in Fig. 4C eingezeichnet.

Die Dichtung 153 ist als Flachdichtung ausgeführt und dichtet den Gehäuseabschnitt 111 gegen die Sensorplatine 140 ab.

In dem durch die Dichtungen 151, 152 und 153 abgedichteten Raum zwischen Sensorplatine 140, Gehäuseabschnitt 111 und Gaseinlass 120 befindet sich demnach ein geschlossenes Volumen, das als Gasausgleichsvolumen V bezeichnet wird. Die Bezeichnung Gasausgleichsvolumen V rührt daher, dass das Gas innerhalb dieses Volumens auf veränderte Gaskonzentrationen außerhalb des Detektors reagieren muss. Erst wenn das Gas innerhalb des Gasausgleichsvolumens V der außen vorherrschenden Konzentration gefolgt ist, kann der EC-Sensor 101 die Gaskonzentration korrekt bestimmen.

Innerhalb des Gasausgleichsvolumens V befindet sich der EC-Sensor 101. Es wurde festgestellt, dass das verbleibende mit Gas gefüllte Restvolumen dennoch in vielen Anwendungen nicht ausreichend gering ist und zu lange Reaktionszeiten des Sensors die Folge waren.

Somit ist in dem Gasausgleichsvolumen V zudem ein Einleger 160 vorgesehen, der einen Großteil des verbleibenden Gases verdrängt. Damit können, wie mit Verweis auf die weiteren Figuren exemplarisch dargestellt wird, Verbesserungen in der Detektionsgenauigkeit und -geschwindigkeit erreicht werden.

Einige EC-Sensoren 101 weisen einen Gaseingang und einen Gasausgang auf, andere lediglich einen Gaseingang, der gleichzeitig auch der Gasausgang ist. Der beispielhaft gezeigte EC-Sensor 101 weist sowohl Gaseingang als auch Gasausgang auf. Der Gasausgang des EC-Sensors 101, also die Lüftungsöffnung 314, ist im Druckequilibirium mit dem Gaseingang, also der Staubabdeckung 306, damit die chemische Reaktion unbeeinflusst abläuft.

Für die Zulassungen im Brandschutz, ggf. sogar Ex-Zulassung, ist es vorteilhaft, dass der Gasausgang des EC-Sensors 101 nicht über eine zweite Melderöffnung mit der Außenatmosphäre verbunden, sondern intern mit dem Gaseingang "kurzgeschlossen" wird. Wie erwähnt ist das Gasausgleichsvolumen V bzw. das gesamte Detektorgehäuse 110 mit Ausnahme der Gas-permeablen Membran 121 geschlossen, es weist also nur eine Öffnung auf.

Hierfür wird wie erwähnt u.a. die Dichtung 152 zwischen EC-Sensor 101 und Gaseinlass 120 mit einer seitlichen Unterbrechung 154 vorgesehen und zwischen EC-Sensor 101 und Gaseinlass 120 befindet sich ein (hier umlaufender) zylindrischer Spalt. Vorstellbar ist hier auch eine Art Längsfüge an der Außenseite des hier beispielhaft zylindrisch ausgeführten EC-Sensors 101.

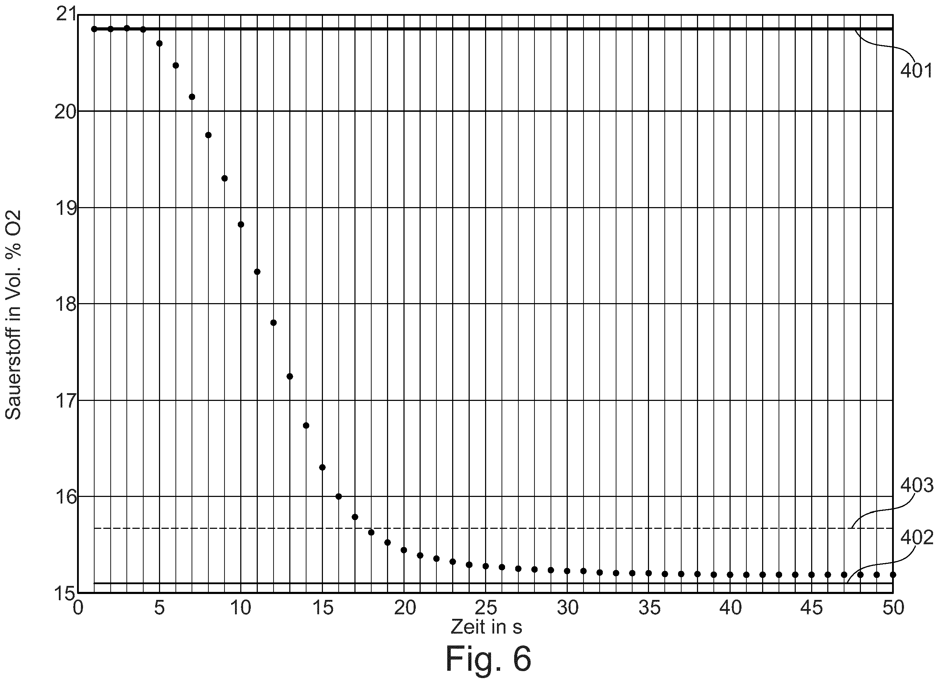

Es ist wie erwähnt eine Erkenntnis der Erfindung, dass bei Begasungstest (Testgas 15% O2) die Zeit bis zum Erreichen des Zielniveaus (T90-Zeit, siehe Figur 6) im komplett zusammengebauten Zustand deutlich größer ist als bei einem teilzusammengebauten Melder ohne Gaseinlass. Aus den Ergebnissen ergaben sich verschiedene Lösungsansätze, zum Erfolg führte letztlich der Ansatz, das freie Volumen im Melder, also das Gasausgleichsvolumen V, zu reduzieren. So wie beschrieben hat der Aufbau ein Gasausgleichsvolumen von etwa 2800 mm³, wobei dieses Innenvolumen auch immer ins O2%-Equilibrium mit der Umgebung gebracht werden muss für eine präzise Messung.

Daraufhin wurde der Einleger 160 entwickelt, welcher zwischen EC-Sensor 101 und Sensorplatine 140 eingesetzt wird. Der Einleger 160 verfügt über drei Öffnungen 161 für die Elektroden 308, 310, 312 und ermöglicht Gasaustausch zwischen dem Gasausgang 314 und letztlich dem Gaseinlass 121 über einen seitlichen, zylindrischen Spalt zwischen EC-Sensor 101 und Einleger 160 mit einer Breite von 0,4 mm. Der Einleger 160 hat einen Mantelkragen 162 und angrenzend an diesen eine Vertiefung 163, die zum Gasaustausch dient.

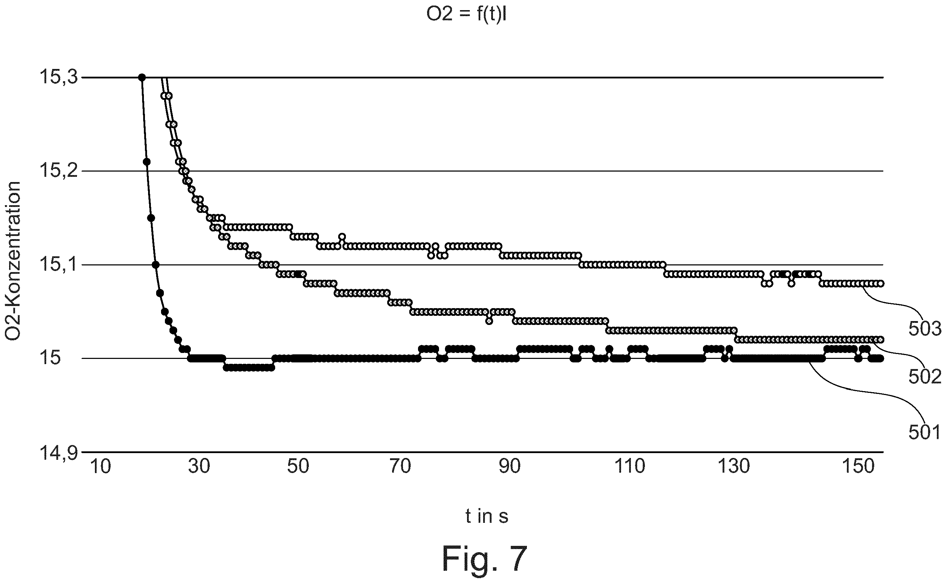

Über die Unterbrechungen 304 der Mandelfläche 302 im Bereich der unteren Stirnfläche 305 des EC-Sensors 101 ist eine Gasverbindung zwischen der Lüftungsöffnung 314 auf der Seite der Elektroden und der Vertiefung 163 hergestellt. Über die Vertiefung 163 und den radialen Spalt zwischen EC-Sensor 101 und Mantelkragen 162 bzw. Gaseinlass 120 wird die Gasverbindung dann bis zu der Dichtung 152 hergestellt. Die Unterbrechung 154 schließt die Gasverbindung dann zu der Membran 121 und damit der Atmosphäre. Der Aufbau mit Einleger 160 hat dann nur noch ein Gasausgleichsvolumen V von ca. 1200 mm³, was die benötigte Zeit zum Erreichen des Equilibriums deutlich verringert (siehe Figur 7).

Darüber hinaus ist der verwendete EC-Sensortemperaturabhängig, aber ungeheizt zu verwenden. Das Ausgabesignal des EC-Sensors wird daher mit einem Temperatursignal eines Temperatursensors auf der Sensorplatine in Nähe zum EC-Sensor korrigiert um ein temperaturunabhängiges Endsignal zu erhalten. Der ungeheizte EC-Sensor ermöglicht einen Betrieb mit nur zwei Drähten. Kann auf den dritten Draht verzichtet werden, sind also nur reine "Datendrähte" erforderlich, könnten auch Reihenschaltungen von mehreren Gasmeldern, oder von Gasmeldern und anderen Komponenten (Rauchmelder, Flammenmelder, ...) genutzt werden, ohne eine besonders aufwändige Verkabelung durch mehrere Heizleitungen zu erfordern.

Bekannt sind im Brandschutz für Reihenschaltungen mehrerer Teilnehmer die nicht-adressierbare Grenzwertlinie und der adressierbare Loop-Ring. Bei beiden wird bei Überschreiten einer im Melder hinterlegten Alarmschwelle ein Alarmsignal erzeugt. Bei der Grenzwertlinie geschieht das in Form einer definierten Stromerhöhung zurück an die Zentrale, wobei jeder ausgelöste Teilnehmer eine Stromerhöhung beiträgt, und keine Rückverfolgung auf den alarmgebenden Teilnehmer möglich ist. Störungen werden mit einer anderen, definierten Stromerhöhung gemeldet. Beim Loop-Ring wird im Melder selbst ein digitales Signal erzeugt, welches den Inhalt der Meldung (Alarm, Störung, ...) und Adresse des Melders umfasst. Es ist grundsätzlich bevorzugt, den Melder auch zu befähigen, eine Gaskonzentration über ein Loop-Protokoll zu übermitteln.

Figur 5 zeigt ein Diagramm einer detektierten O2-Konzentration über die Zeit. Die T90 Zeit ist ein Parameter der Aufschluss darüber gibt, wie schnell bei einer Konzentrationsänderung eine neue Konzentration detektiert wird.

Zur Ermittlung der T90 Zeit wurde ein Melder bei -26 °C in einer Umgebung mit etwa 23 Vol. % O2 betrieben und nach dem Einlaufen mit einem Prüfgas mit ca. 15 Vol. % O2 in N2 begast. Die Messwerte wurden gespeichert, bis der Melder einen stabilen Endwert erreicht hat. Aus den Daten wurde der Startwert (Gerade 401 in Figur 6) und der Endwert (Gerade 402 in Figur 6) ermittelt.

Anschließend wurde der Wert für 90 % der Signaländerung berechnet (Gerade 403 in Figur 6) und die Zeit bis dieser Wert erreicht wurde aus den Messdaten abgelesen.

Die ermittelte T90 Zeit beträgt 18 s.

Figur 7 zeigt den Verlauf detektierter O2-Konzentrationen gleicher Gasdetektoren verschiedener Ausführungen über die Zeit. Die Ausgangskonzentration der Umgebung in der gemessen wurde betrug ungefähr 20,8 Vol. % O2, also eine Sauerstoffkonzentration von ungefähr 20,8%. Die Gasdetektoren wurden dann mit einem Prüfgas mit ca. 15 Vol. % O2 in N2 begast.

Graph 501 zeigt den Messverlauf eines Gasdetektors ohne Gaseinlass. Graph 502 zeigt den Messverlauf eines Gasdetektors mit Gaseinlass und Einlage. Graph 503 zeigt den Messverlauf eines Gasdetektors mit Gaseinlass ohne Einlage.

Aus dem Diagramm ist ersichtlich, dass die T95 Zeiten (ungefähr 15,3%) nur unwesentlich abweichen. Selbiges trifft auf die T90 Zeiten zu, die in Figur 7 nicht gezeigt sind.

Anders sieht es aus für die T99 Zeiten (ungefähr 15,1%) aus, die mit Einlage sehr viel besser sind. Der Gasdetektor mit Gaseinlass mit Einlage 502 weist eine T99 Zeit von etwa 40 Sekunden auf, während der Gasdetektor mit Gaseinlass ohne Einlage 503 eine T99 Zeit von etwa 115 Sekunden aufweist.

Der Gasdetektor mit Einlage liefert also schneller genauere Messwerte als der sonst baugleiche Gasdetektor ohne Einlage.

Im praktischen Einsatz ist ein Gasdetektor ohne Gaseinlass nicht umsetzbar, da schon ein Auftreffen von Staub und ähnlichem auf der Oberfläche des EC-Sensors die Messergebnisse verfälschen und ggf. sogar unmöglich machen.

### Bezugszeichen:

- 100: Gasdetektor
- 101: EC-Sensor
- 110: Detektorgehäuse
- 111: Gehäuseabschnitt
- 112: Gehäusesockel
- 114: Dichtung
- 115: Aufnahme
- 120: Gaseinlass
- 121: Membran
- 122: Antriebsprofil
- 124: Anzeigebereich
- 125: LED Steuerelektronik
- 126: LED
- 130: Befestigungsmittel
- 132: Konfigurationsmittel
- 134: Befestigungsmittel
- 136: Befestigungsmittel
- 138: Befestigungsmittel
- 139: Umwandlungselektronik
- 140: Sensorplatine
- 141: Gasdetektorplatine
- 142: Abdeckplatte
- 143: Konfigurationsmittel
- 144: Aussparung
- 146: Konnektor
- 148: Konnektor
- 150: Kabeleinführung
- 151: O-Ring
- 152: zylindrische Dichtung
- 153: Flachdichtung
- 154: Unterbrechung
- 160: Einleger
- 161: Öffnungen
- 162: Mantelkragen
- 163: Vertiefung
- 302: Mantelfläche
- 303: obere Stirnfläche
- 304: Unterbrechungen
- 305: untere Stirnfläche
- 306: Staubabdeckung
- 308: Referenzelektrode
- 310: Arbeitselektrode
- 312: Gegenelektrode
- 314: Lüftungsöffnung
- 401: Startwert
- 402: Endwert
- 403: Wert für 90% der Signaländerung
- 501: Messkurve Gasdetektor ohne Gaseinlass
- 502: Messkurve Gasdetektor mit Gaseinlass mit Einlage
- 503: Messkurve Gasdetektor mit Gaseinlass ohne Einlage

## Patentansprüche

1. Verfahren zur Detektion einer Gaskonzentration in einem Gasdetektor (100), umfassend
- Bereitstellen eines elektrochemischen Sensors (101), EC-Sensor, der zur Detektion einer Konzentration wenigstens eines Gases oder Gasgemischs ausgebildet ist, wobei der EC-Sensor (101) mehrere Elektroden (308, 310, 312), die auf einer Seite des EC-Sensors (101) ausgebildet sind, und einen Gaseingang (306) aufweist,
- Verbinden einer Sensorplatine (140) mit den mehreren Elektroden (308, 310, 312) des EC-Sensors (101), und
- Umschließen des EC-Sensors (101) und der Sensorplatine (140) mit einem Detektorgehäuse (110), wobei das Detektorgehäuse (110) bis auf einen Gaseinlass (120) mit einer Einlassöffnung (121) geschlossen ist,
wobei das Detektorgehäuse (110) ein Gasausgleichsvolumen (V) im Inneren zwischen Detektorgehäuse (110) und EC-Sensor (101) definiert, das eine hinreichende Gasströmung zu dem Gaseingang (306) des EC-Sensors (101) ermöglicht.

2. Gasdetektor (100), der aufweist:
- einen elektrochemischen Sensor (101), EC-Sensor, der zur Detektion einer Konzentration wenigstens eines Gases oder Gasgemischs ausgebildet ist, wobei der EC-Sensor (101) mehrere Elektroden (308, 310, 312), die auf einer Seite (305) des EC-Sensors (201, 301) ausgebildet sind, und einen Gaseingang aufweist,
- eine Sensorplatine (140), die in Verbindung mit den mehreren Elektroden des EC-Sensors (101) steht, und
- ein Detektorgehäuse (110), das den EC-Sensor (101) und die Sensorplatine (140) umschließt,
wobei das Detektorgehäuse (110) einen Gaseinlass (120) mit einer Einlassöffnung (121) aufweist,
wobei das Detektorgehäuse (110) ein Gasausgleichsvolumen (V) im Inneren zwischen Detektorgehäuse (110) und EC-Sensor (101) definiert, das eine Gasströmung zu dem Gaseingang des EC-Sensors (101) ermöglicht.

3. Gasdetektor (100) nach Anspruch 2, wobei der EC-Sensor (101) einen Gasauslass auf der dem Gaseingang gegenüberliegenden Seite aufweist, wobei eine Gasströmung ausgehend von dem Gaseinlass (120) des Detektorgehäuses (110) durch den EC-Sensor (101) von dem Gaseingang zu dem Gasauslass und zurück zu dem Gaseinlass (120) des Detektorgehäuses (110) in einem Luftspalt zwischen Detektorgehäuse (110) und EC-Sensor (101) ausgebildet ist.

4. Gasdetektor (100) nach einem der vorstehenden Ansprüche, wobei der EC-Sensor (101) eine im Wesentlichen zylindrische Form aufweist, wobei die Elektroden und der Gaseingang des EC-Sensor (101) auf jeweils gegenüberliegenden Stirnflächen der zylindrischen Form ausgebildet sind.

5. Gasdetektor (100) nach Anspruch 4, wobei das Detektorgehäuse (110) einen axial entlang des Zylinders verlaufenden Luftspalt radial außerhalb zwischen Zylinder des EC-Sensors (101) und Detektorgehäuse (110 definiert.

6. Gasdetektor (100) nach einem der vorstehenden Ansprüche, der weiter einen Einleger (160) aufweist, der zwischen EC-Sensor (101) und Sensorplatine (140) angeordnet ist, derart, dass das von Luft eingenommene Volumen des Gasausgleichsvolumens zwischen EC-Sensor (101) und Sensorplatine (140) verkleinert ist, wobei insbesondere der Abstand zwischen EC-Sensor (101) und Sensorplatine (140) durch die Elektroden definiert ist.

7. Gasdetektor (100) nach einem der vorstehenden Ansprüche, der weiter eine Gasdetektorplatine (141) aufweist, wobei die Gasdetektorplatine (141) innerhalb des Detektorgehäuse (110) und in Kommunikationsverbindung mit der Sensorplatine (140) angeordnet ist, wobei die Gasdetektorplatine (141) dazu ausgebildet ist, ein Gasdetektionssignal von der Sensorplatine (140) zu empfangen und in Abhängigkeit des Gasdetektionssignals mit einer weiteren Einheit, insbesondere einer Brandmelder- und/oder Löschsteuerzentrale, zu kommunizieren.

8. Gasdetektor (100) nach einem der vorstehenden Ansprüche, wobei das Detektorgehäuse (110) einen Gehäusesockel (112), der insbesondere an einer Wand verschraubbar ausgestaltet ist, und einen darauf montierbaren, insbesondere daran verschraubbaren, Gehäuseabschnitt (111) aufweist, wobei der Gehäuseabschnitt (111) insbesondere eine Aufnahme (115) für ein Belüftungselement mit dem Gaseinlass (120) des Detektorgehäuses (110) an einer dem Gehäusesockel (112) gegenüberliegenden Seite, insbesondere mittig an der dem Gehäusesockel (112) gegenüberliegenden Seite, aufweist.

9. Gasdetektor (100) nach Anspruch 8, wobei das Detektorgehäuse (110) mit Ausnahme der Aufnahme (115) für das Belüftungselement einen dichten Aufnahmeraum für den EC-Sensor (101) definiert.

10. Gasdetektor (100) nach einem der vorstehenden Ansprüche, wobei der EC-Sensor (101) ein analoges Stromsignal zwischen zwei der Elektroden als Detektionssignal, insbesondere eine Spannung im nV-Bereich, bereitstellt und/oder wobei der Gaseinlass (120) eine Gas-permeable Membran (121) aufweist und/oder wobei der Gasdetektor (100) nicht zur Beheizung des EC-Sensors (101) ausgebildet ist.

11. Gasdetektor (100) nach einem der vorstehenden Ansprüche, wobei der Gasdetektor (100) einen Temperatursensor aufweist und ein Detektionswert des EC-Sensors (101) mittels eines von dem Temperatursensor erfassten Wertes korrigiert wird.

12. Gasdetektor (100) nach einem der vorstehenden Ansprüche, wobei der EC-Sensor (101) in dem Detektorgehäuse (110) mit mehreren Dichtungen (151, 152, 153) eingefasst ist, wobei die Dichtungen insbesondere:
- eine zylindrische Flachdichtung (153), die an der Sensorplatine (140) radial nach außen abdichten, und/oder
- einen O-Ring (151) zwischen Sensorplatine (140) bzw. Flachdichtung und Gaseinlass (120), und/oder
- eine zylindrische Dichtung (152) mit seitlicher Unterbrechung (154) am vorderen Ende (303) des EC-Sensors (101) einen Gaseingang (306) des EC-Sensors (101) umschließend aufweisen.

13. Gasdetektor (100) nach einem der vorstehenden Ansprüche, wobei ein Gasauslass des EC-Sensors (101) im Druckequilibrium mit dem Gaseingang des EC-Sensors (101) ist.

14. Gasdetektor (100) nach einem der vorstehenden Ansprüche, wobei das Gasausgleichsvolumen zwischen Detektorgehäuses (110, 210) und EC-Sensor (101) höchstens 2.000 mm³, vorzugsweise höchstens 1.700 mm³ und besonders bevorzugt höchstens etwa 1.200 mm³ beträgt.

15. Gasdetektor (100) nach einem der vorstehenden Ansprüche, wobei der Gasdetektor (100) über genau zwei Anschlussleitungen mittels einer Grenzwertlinie und/oder einem Loop-Ring und/oder eine 4-20mA Zweidrahtleitung mit einer Brandmelder- und/oder Löschsteuerzentrale verbindbar ist.
